# DEMANDE DE BREVET EUROPEEN

(11) **EP 1 493 422 A1**
(43) Date de publication de la demande: **05.01.2005**
(21) Numéro de dépôt: 04300415.9
(22) Date de dépôt: 29.06.2004
(51) Int. Cl.: A61K 7/02

(54) **Composition cosmétique comprenant au moins un organopolysiloxane hydrophile, au moins une huile hydrocarbonée et au moins un ester hydrocarboné court**

(30) Priorité: 30.06.2003 FR 0307873
(71) Demandeur: L'OREAL, 75008 Paris (FR)
(72) Inventeur: Blin, Xavier, 75015 Paris (FR)
(74) Mandataire: Le Coupanec, Pascale

(57) **Abrégé**

La présente invention concerne une composition cosmétique associant au moins un organopolysiloxane possédant au moins un radical hydrophile à au moins une huile hydrocarbonée non compatible avec ledit organopolysiloxane caractérisée en ce qu'elle comprend en outre à titre d'agent compatibilisant une quantité efficace d'au moins un ester hydrocarboné comprenant moins de 40 atomes de carbone.

## Description

La présente invention concerne une composition cosmétique contenant au moins un organopolysiloxane comportant un motif hydrophile, une huile hydrocarbonée non compatible avec ledit organopolysiloxane et un agent compatibilisant.

Au sens de la présente invention, les compositions cosmétiques constituent notamment, des compositions de maquillage, comme des poudres, des fards à paupières, des fonds de teint, des anti-cernes, des rouges à lèvres, des produits de maquillage pour le corps, des mascaras, des eye-liners ou encore des compositions de soin de la peau.

L'utilisation d'huiles hydrocarbonées associées à des dérivés organopolysiloxanes dans des compositions cosmétiques, notamment de maquillage, est connue. Par exemple, il est décrit dans la demande WO 03/000223 des émulsions de type polyol dans silicone. La demande de brevet US 2001/0051686 décrit une composition de type émulsion eau-dans-huile dont la phase huileuse est composée d'au moins une huile hydrocarbonée, pouvant contenir une huile siliconée en faible quantité. Dans le cas de poudres cosmétiques par exemple, un mélange d'huiles minérale et végétale associées à des esters d'acides gras est généralement utilisé à titre de liant, pour obtenir des compositions adhérant bien sur la peau et résistantes aux chocs. Quant aux dérivés organopolysiloxane, ils sont généralement incorporés dans ce type de liant notamment pour apporter de la douceur. Ils permettent également d'améliorer la tenue des compositions de fonds de teint ou de rouges à lèvres par exemple, et d'obtenir sur les matières kératiniques un film particulièrement homogène et possédant de bonnes propriétés cosmétiques.

Ainsi, il est intéressant de disposer de compositions cosmétiques comprenant à la fois des dérivés organopolysiloxanes et des huiles hydrocarbonées.

Toutefois, un nombre important de composés siliconés s'avère incompatible avec les huiles hydrocarbonées conventionnelles. Cette incompatibilité peut se manifester notamment par des phénomènes de déphasage, de relargage ou encore de synérèse lorsque ces deux types de composés sont mis en présence. Il s'en suit donc un problème d'hétérogénéité au niveau de la composition. Ce problème d'incompatibilité est particulièrement préjudiciable dans le cas des poudres cosmétiques dans la mesure où il empêche d'obtenir une bonne dispersion des pigments.

Diverses solutions ont déjà été proposées pour tenter de suppléer cette incompatibilité. Ainsi, le document EP 0 548 694 propose de mettre en oeuvre dans une composition cosmétique un organopolysiloxane modifié par des polyoxyalkènes. Les documents EP 1 112 734, EP 0 967 250 et WO 97/16157 décrivent des compositions cosmétiques dans lesquelles l'huile hydrocarbonée et le dérivé siliconé considéré sont associés notamment à un solvant volatil du type silicone linéaire ou cyclique et composés paraffiniques. Les compositions cosmétiques correspondantes sont décrites comme présentant une meilleure tenue vis-à-vis de la couleur, du transfert et de la migration.

Toutefois, l'utilisation de ce type de solvant volatil soulève à son tour quelques problèmes. Tout d'abord, il ne s'avère pas toujours suffisamment inerte vis-à-vis des matériaux plastiques constituant les articles de conditionnement des compositions correspondantes. D'autre part, il n'est pas toujours compatible avec un usage dans les formulations cosmétiques dont la préparation nécessite une étape de chauffage. Enfin, l'incorporation d'un solvant volatil nuit généralement aux propriétés de brillance et de confort des compositions cosmétiques correspondantes. En particulier, le séchage rapide de la formule appliquée provoque fréquemment une sensation de dessèchement au niveau de la zone maquillée.

Il subsiste donc un besoin d'une composition ne présentant pas les inconvénients évoqués ci-dessus, c'est-à-dire qui soit homogène et stable, dotée de bonnes propriétés de non transfert et de non migration, ne desséchant pas et ne tiraillant pas la peau ou les lèvres sur lesquelles elle est appliquée.

De manière inattendue, les inventeurs ont constaté qu'il était possible de satisfaire à l'ensemble de ces exigences, sous réserve d'associer à ces deux types de composés, un agent compatibilisant spécifique.

Plus précisément, la présente invention concerne, selon l'un de ses aspects, une composition cosmétique anhydre associant au moins un organopolysiloxane possédant au moins un radical hydrophile à au moins une huile hydrocarbonée non compatible avec ledit organopolysiloxane caractérisée en ce qu'elle comprend en outre à titre d'agent compatibilisant une quantité efficace d'au moins un ester hydrocarboné comprenant moins de 40 atomes de carbone.

Selon une variante privilégiée de l'invention, cet ester est non volatil.

L'invention concerne encore, selon un autre de ses aspects, un procédé cosmétique de soin ou de maquillage des lèvres, des phanères ou de la peau, comprenant l'application sur les lèvres, les phanères ou la peau d'une composition cosmétique conforme à l'invention.

Elle a également pour objet, selon un autre de ses aspects, l'utilisation d'une composition conforme à l'invention pour obtenir un maquillage de la peau, des lèvres ou des phanères.

Les inventeurs ont ainsi mis en évidence qu'en associant un organopolysiloxane conforme à l'invention, une huile hydrocarbonée non compatible avec ledit organopolysiloxane, et un ester hydrocarboné comportant moins de 40 atomes de carbones, il est possible d'obtenir une composition cosmétique, qui est homogène durablement et présente une excellente cohésion, mais également permet d'obtenir un dépôt brillant, de bonne tenue et ne migrant pas.

L'ester selon l'invention est particulièrement intéressant dans la mesure où il permet de réduire significativement la quantité de solvant volatil, conventionnellement utilisée, notamment à une valeur inférieure à 10 % en poids, en particulier inférieure ou égale à 5 % en poids, voire de s'en affranchir totalement dans une composition cosmétique conforme à la présente invention.

Au sens de la présente invention, on entend :
- par « huile », tout milieu non aqueux, liquide, à température ambiante (25 °C) et à pression atmosphérique (760 mm Hg),
- par « ester hydrocarboné», un composé comprenant au moins une fonction ester,
- par « organopolysiloxane », un polymère comprenant un squelette polymérique composé de motifs répétitifs siloxy qui peuvent être des motifs, cycliques, linéaires ou ramifiés, par exemple des motifs dialkyle inférieurs siloxy comme notamment des motifs diméthylsiloxy.
- par « organopolysiloxane hydrophile », un organopolysiloxane possédant au moins un radical hydrophile, le radical hydrophile étant greffé à l'une des extrémités du squelette organopolysiloxane ou sur le squelette organopolysiloxane.
- par « radical hydrophile », on entend un radical qui confère des propriétés hydrophiles à l'organopolysiloxane. Des exemples de radicaux hydrophiles sont hydroxy, polyéthylèneoxy, hydroxyle, carboxylate, sulfonate, sulfate, phosphate et amine.
- par composé ou milieu « non volatil », tout composé ou milieu susceptible de rester sur la peau, les lèvres ou les phanères pendant plusieurs heures. Un milieu non volatil a en particulier une pression de vapeur, non nulle, inférieure à 0,02 mm de mercure (2,66 Pa), à température ambiante (25 °C) et pression atmosphérique (760 mm Hg),
- par "non compatible", on entend désigner un état d'incompatibilité se manifestant par la présence d'un trouble ou d'un déphasage visible à l'oeil nu dans un tube à essai de 1 à 2 cm de diamètre contentant l'huile hydrocarbonée à 60 °C lorsque l'organopolysiloxane y est introduite à l'échelle d'une dizaine de %.

Les esters conformes à l'invention, qui sont plus particulièrement non volatils, peuvent être des monoesters, des diesters ou des polyesters et sont plus particulièrement des monoesters c'est-à-dire portant une unique fonction ester. Ces esters peuvent être linéaires, ramifiés ou cycliques, saturés ou insaturés. De préférence, ils sont ramifiés et saturés.

En particulier, les esters hydrocarbonés peuvent répondre à la formule RCOOR' dans laquelle RCOO représente un reste d'acide gras comportant de 2 à 28 atomes de carbone, et R' représente une chaîne hydrocarbonée contenant de 1 à 28 atomes de carbone. Plus particulièrement les groupements R et R' sont tels que l'ester correspondant est non volatil.

Ces esters non volatils peuvent notamment être en C₁₀ à C₂₅ et en particulier en C₁₄ à C₂₂. Ils peuvent être choisis parmi des esters des acides en C₂ à C₁₈ et notamment, des alcools en C₂ à C₂₀ ou de polyols en C₂ à C₈ ou de leurs mélanges.

Ainsi, les esters peuvent être choisis parmi une liste non limitative comprenant les esters de l'acide néopentanoïque comme le néopentanoate d'isodécyle, le néopentanoate d'isotridécyle, le néopentanoate d'isostéaryle, le néopentanoate d'octyldocécyle, les esters de l'acide isononanoïque comme l'isononanoate d'isononyle, l'isononanoate d'octyle, l'isononanoate d'isodécyle, l'isononanoate d'isotridécyle, l'isononanoate d'isostéaryle, mais aussi les esters de l'alcool isopropylique, tels que le myristate d'isopropyle, le palmitate d'isopropyle, le stéarate ou l'isostéarate d'isopropyle, l'octanoate de cétyle, l'octanoate de tridécyle, le 4-diheptanoate et le palmitate d'éthyle 2-hexyle, le benzoate d'alkyle, le diheptanoate de polyéthylène glycol, le diéthyl 2-d'hexanoate de propylèneglycol et leurs mélanges. Ledit ester peut être également choisi parmi les esters de synthèse notamment d'acide gras comme l'huile de purcellin, le myristate d'isopropyle, le palmitate d'éthyle, le stéarate d'octyle ; les esters hydroxylés comme le lactate d'isostéaryle, l'hydroxystéarate d'octyle, l'adipate de diisopropyle, les heptanoates, octanoates, décanoates d'alcool gras et leurs mélanges.

L'isononanoate d'isononyle convient tout particulièrement à la réalisation de l'invention.

Cet ou ces ester(s) hydrocarboné(s) peut(peuvent) être utilisé(s) dans la composition à raison de 5 à 90 %, notamment de 10 à 60 % et en particulier de 20 à 50 % en poids par rapport au poids total de la composition.

L'organopolysiloxane hydrophile utilisé dans l'invention peut être liquide ou solide à température ambiante.

Le radical hydrophile peut notamment être un groupement hydrocarboné, saturé ou insaturé, comprenant éventuellement un ou plusieurs hétéroatomes comme par exemple l'oxygène ou le soufre, lequel groupement hydrocarboné peut être substitué par au moins un groupement hydrophile. Des exemples de groupements hydrophiles sont notamment des fonctions hydroxyle, carboxylique, carboxylate, thiol, amine, sulfonate, sulfate, phosphate et/ou hydroxypolyéthylènoxy.

Plus précisément, le radical hydrophile peut répondre à la formule (II) : 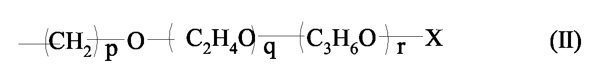 dans laquelle
- p varie de 0 à 5, q varie de 0 à 100, r varie de 0 à 50, avec p ou q étant différent de zéro,
- les unités (C₂H₄O) et (C₃H₆O) peuvent être réparties aléatoirement ou par blocs, et
- X est un hydrogène ou un radical alkyle en C₁-C₁₀ le cas échéant substitué par un ou plusieurs fonctions de type hydroxyle, thiol, amine, carboxylique, carboxylate, amide, phosphate, sulfate et sulfonate.

En particulier, p peut varier de 1 à 5, q de 1 à 100 et r de 1 à 50. X peut plus particulièrement figurer un atome d'hydrogène.

En particulier, l'organopolysiloxane, selon l'invention, peut comprendre à titre de radical hydrophile au moins un radical hydroxy-polyalkylèneoxy et notamment un radical hydroxy-polyéthylèneoxy.

Notamment, l'organopolysiloxane selon l'invention peut répondre à la formule (I) : 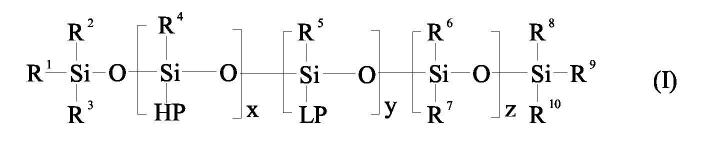 dans laquelle
- R¹, R², R³, R⁴, R⁵, R⁶ R⁷, R⁸, R⁹ et R¹⁰ représentent indépendamment l'un de l'autre un radical alkyle en C₁ à C₆, linéaire, ramifié ou cyclique, saturé ou non,
- HP est un radical portant au moins un groupement hydrophile tel que défini précédemment,
- LP est un radical lipophile, et
- x varie de 1 à 5000 ; y de 0 à 5000 ; z de 0 à 5000.

En ce qui concerne le radical LP, il peut notamment être choisi parmi des alkyles en C₁ à C₄₀ ramifiés, cycliques ou linéaires, des atomes de fluors, des radicaux aryles, aryloxy, des acyles d'hydrocarbyles en C₁ à C₄₀ et des hydroxypropylénoxy.

Selon une variante particulière de l'invention, l'organopolysiloxane appartient à la famille des diméthicones-polyéthylèneglycols et notamment peut être choisi dans le groupe comprenant les diméthicones copolyol, en particulier la cétyldiméthicone copolyol et leurs dérivés. L'organopolysiloxane hydrophile selon la présente invention peut être le produit commercialisé sous la marque Abil WE09 ou Abil EM90 par la société Degussa-Goldschmidt. L'organopolysiloxane hydrophile selon la présente invention peut être également le produit commercialisé sous la référence KF-6017 par la société Shin-Etsu.

Le composé organopolysiloxane peut être en tout ou partie fluoré. En particulier, les groupements di-alkyles inférieurs siloxy peuvent être substitués par un ou plusieurs atomes de fluor.

L'organopolysiloxane peut être présent à raison de 0,1 à 50 %, notamment de 0,5 à 40 %, et en particulier de 1 à 30 % en poids par rapport au poids total de la composition.

Sa viscosité peut être comprise entre 0,5 et 1 000 000 cst, notamment 25 à 600 000 est et en particulier 200 000 à 250 000 est, à 25 °C mesurée selon la norme ASTM D-445.

L'huile hydrocarbonée, utilisable selon l'invention, peut être apolaire et/ou non volatile. Elle est non compatible avec l'organopolysiloxane, en l'absence de l'ester conforme à l'invention.

Elle peut être choisie parmi les huiles d'origine animale, telles que le perhydrosqualène, les huiles hydrocarbonées végétales telles que les triglycérides liquides d'acide gras de 4 à 24 atomes de carbone comme les triglycérides heptanoïques et octanoïques, les huiles de tournesol, de maïs, de soja, de courge, de pépins de raisin, de sésame, de colza, de noisette, d'abricot, de macadamia, de ricin, d'avocat, les triglycérides des acides capryliques/capriques, l'huile de jojoba, le beurre de karité ; les hydrocarbures linéaires ou ramifiés d'origine minérale ou synthétique, tels que les huiles de paraffines et leurs dérivés, la vaseline, les polydécènes, les polyisobutènes hydrogénées, les huiles hydrocarbonées et/ou siliconées partiellement fluorées et leurs mélanges.

Elle peut être plus particulièrement choisie parmi les paraffines hydrocarbonées non volatiles, hydrogénées ou non, telles que le polydécène, le polyisobutène, les huiles minérales, le squalène et les polyalphaoléfines liquides, et notamment être une isoparaffine du type polyisobutène hydrogénée comme celle commercialisée sous la dénomination Parléam.

L'huile hydrocarbonée peut être présente à raison de 3,5 à 50 %, notamment de 4 à 40 % et en particulier de 4 à 35 % en poids par rapport au poids total de la composition.

D'une manière générale, les quantités respectives conformes à l'invention d'organopolysiloxane hydrophile, d'huile hydrocarbonée et d'ester hydrocarboné sont choisies en quantité suffisante pour conférer à la composition les propriétés en terme de stabilité, homogénéité, non-transfert, non migration, brillance et confort attendues.

Plus particulièrement, le composé organopolysiloxane peut être présent en une proportion massique égale ou inférieure à celle de l'huile hydrocarbonée. En d'autres termes, le rapport pondéral R défini par :

R = % massique de l'organopolysiloxane / % massique d'huile hydrocarbonée est inférieur à 1, notamment varie de 0,01 à 0,7 et en particulier de 0,1 à 0,5.

Selon une variante particulière de l'invention, la composition associe une diméthicone copolyol, du polyisobutène hydrogéné et notamment celle commercialisée sous le nom Parléam par la société Nippon Oil Fats, et de l'isononanoate d'isononyle

La composition peut contenir en outre, au moins un corps gras différent de l'organopolysiloxane polaire, de l'huile hydrocarbonée non compatible et de l'ester hydrocarbonée. Ce corps gras peut notamment être choisi parmi les cires, les corps gras pâteux à température ambiante, les huiles cosmétiquement et dermatologiquement acceptables et leurs mélanges.

La nature et la quantité des cires et des corps gras pâteux sont généralement ajustées en fonction des propriétés mécaniques et de textures recherchées.

Le ou les corps gras additionnels de la composition peuvent représenter de 0,1 % à 90 % du poids total de la composition, de préférence de 5 à 60 % et mieux de 10 à 50%.

De préférence, la composition de l'invention contient peu d'huiles volatiles et notamment moins de 10 % du poids total de la composition, de préférence moins de 5 %, mieux moins de 2 % et en particulier est exempte d'huile volatile.

La composition, selon l'invention, peut contenir en outre, au moins une phase particulaire inerte et notamment une charge en particulier inerte, absorbante ou non.

Cette phase particulaire inerte peut représenter de 0,1 à 30 % et mieux de 2 à 25 % et encore mieux de 10 à 20 % en poids du poids total de la composition.

La composition de l'invention peut également comprendre au moins une matière colorante comme par exemple un ou plusieurs composés pulvérulents et/ou un ou plusieurs colorants liposolubles, notamment à raison de 0 à 70 % du poids total de la composition et notamment de 0,01 à 70 %. Le ou les composés pulvérulents peuvent être choisis parmi les pigments, les nacres, habituellement utilisés dans les compositions cosmétiques ou dermatologiques et leurs mélanges. Les composés pulvérulents colorants peuvent représenter jusqu'à 50 % du poids total de la composition, par exemple de 0,01 à 50 % et mieux de 1 à 40 % en poids par rapport au poids total de la composition.

La composition de l'invention peut, en outre, contenir un ou plusieurs actifs cosmétiques ou dermatologiques tels que ceux classiquement utilisés.

Comme actifs cosmétiques ou dermatologiques, utilisables dans la composition de l'invention, on peut citer les hydratants, vitamines, acides gras essentiels, sphingolipides, filtres solaires, agents apaisants (Bisabolol, par exemple). Ces actifs sont utilisés en quantité habituelle pour l'homme du métier et notamment à des concentrations de 0 à 20 % et notamment de 0,001 à 20 % et mieux de 0,1 à 5 % en poids par rapport au poids total de la composition. La composition selon l'invention peut, de plus, comprendre, selon le type d'application envisagée, les constituants classiques utilisés dans les domaines considérés, qui sont présents en une quantité appropriée à la forme galénique souhaitée.

La composition peut comprendre, en outre, tout additif usuellement utilisé dans de telles compositions, tel que des épaississants (hectorite modifiée par chlorure de distéaryl di-méthyl ammonium par exemple connue sous le nom de Bentone®), des antioxydants, des parfums, des conservateurs, des tensioactifs, des polymères liposolubles. Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

Les compositions selon l'invention peuvent être préparées de manière usuelle par l'homme du métier. Elles peuvent se présenter sous forme d'un produit coulé et par exemple sous la forme d'un stick ou bâton, ou sous la forme de coupelle utilisable par contact direct ou à l'éponge ou encore dans une bouillotte. En particulier, elles trouvent une application en tant que fond de teint, fard à joues ou à paupières coulé, rouge à lèvres, base ou baume de soin pour les lèvres, produit anti-cernes, d'un eye-liner et/ou d'un mascara. Elles peuvent aussi se présenter sous forme d'une pâte souple ou encore de gel, de crème plus ou moins fluide. Elles peuvent alors constituer des fonds de teint ou des rouges à lèvres, des brillants à lèvres (gloss en terminologie anglo-saxonne), des produits solaires ou de coloration de la peau.

Les compositions de l'invention sont avantageusement anhydres, c'est à dire peuvent contenir moins de 5 %, en particulier moins de 2 %, et plus particulièrement moins de 1 % en poids d'eau, par rapport au poids total de la composition. Elles peuvent alors se présenter notamment sous forme de gel huileux, de liquide huileux, de pâte ou de stick ou encore sous forme de dispersion vésiculaire contenant des lipides ioniques et/ou non ioniques.

Elles peuvent aussi se présenter sous forme d'une émulsion simple ou multiple à phase continue huileuse ou aqueuse, de dispersion huileuse dans une phase aqueuse grâce à des vésicules contenant des lipides ioniques et/ou non ioniques. Il peut aussi s'agir d'émulsion eau-dans-huile ou huile-dans-eau. Selon un mode particulier de réalisation, lorsque les compositions cosmétiques conformes à la présente invention sont sous la forme d'émulsions, celles-ci ne sont pas de type polyol/silicone.

Ces formes galéniques sont préparées selon les méthodes usuelles des domaines considérés.

Les compositions à application topique peuvent constituer notamment une composition cosmétique ou dermatologique, de protection, de traitement ou de soin pour le visage, pour le cou, pour les mains ou pour le corps (par exemple crème de soin, huile solaire, gel corporel), une composition de maquillage (par exemple gel de maquillage, crème, stick) ou une composition de bronzage artificiel ou de protection de la peau.

En particulier, une composition selon l'invention peut se présenter sous la forme d'un fond de teint, d'un fard à joues ou à paupières, d'un rouge à lèvres, d'une base ou baume de soin pour les lèvres, d'un produit anti-cernes, d'un eye-liner et/ou d'un mascara.

La composition selon l'invention peut être utilisée dans un procédé de soin de lèvres, de la peau ou des phanères consistant à appliquer une telle composition sur les lèvres, la peau ou les phanères.

La composition peut être utilisée pour obtenir un maquillage de la peau, des lèvres ou des phanères.

L'invention est illustrée plus en détail dans les exemples suivants. Les pourcentages sont des pourcentages massiques. Les noms de certains ingrédients sont donnés en nom CTFA.

### EXEMPLE 1

Cet exemple démontre l'efficacité d'un ester conforme à l'invention à titre d'agent compatibilisant.

Dans une phase grasse hydrocarbonée à base de polyisobutène hydrogéné (vendue sous la référence Parléam par Nippon Oil Fats) présente à raison de 60 % en poids de la composition, on disperse 10 % en poids d'une silicone fluorée éthoxylée (diméthicone copolyol) (commercialisé sous la référence KF6017 par Shin-Etsu) en présence de 30 % en poids d'isonanoate d'isononyle. On obtient un mélange homogène stabilisé transparent et incolore.

Lors de la reproduction de ce même mélange en absence d'isononanoate d'isononyle, on observe à chaud (60 °C) la formation d'un trouble puis d'un déphasage.

### EXEMPLE 2

Une formulation stick de rouge à lèvres de composition suivante a été préparée en utilisant en partie les composés de l'exemple 1.

| *Phase A* : | % en poids |
|---|---|
| Parleam | 36 |
| Isononanoate d'isononyle | 30 |
| Cire de polyéthylène (PM 500) | 15 |
| Diméthicone copolyol | 10 |

| *Phase B* : | |
|---|---|
| Pigments | 8,8 |

| *Phase C* : | |
|---|---|
| Parfum | 0,2 |

Le stick brillant obtenu présente une tenue améliorée en présence de l'association ester et tensioactif, et en particulier une migration diminuée.

### EXEMPLE 3

La formulation de l'exemple 2 a été testée en comparaison avec une formulation ne contenant pas l'association diméthicone copolyol et l'isononanoate d'isononyle.

Les résultats de tenue en migration sont présents ci-après en tableau I

| FORMULE | EXEMPLE 2 | TEMOIN |
|---|---|---|
| Filage dans les ridules après 1 heure | 2,0 | 5,6 |

On note l'amélioration importante en terme de migration. La formulation, conforme à l'invention, ne présente pratiquement pas de filage dans les ridules à 1 heure comparativement à la formulation des témoins.

## Revendications

1. Composition cosmétique anhydre associant au moins un organopolysiloxane possédant au moins un radical hydrophile à au moins une huile hydrocarbonée non compatible avec ledit organopolysiloxane **caractérisée en ce qu'**elle comprend en outre à titre d'agent compatibilisant une quantité efficace d'au moins un ester hydrocarboné comprenant moins de 40 atomes de carbone.

2. Composition selon la revendication 1, **caractérisée en ce que** l'ester hydrocarbonée est non volatil.

3. Composition selon la revendication 1 ou 2, **caractérisée en ce que** l'ester est un monoester.

4. Composition selon la revendication 1, 2 ou 3, **caractérisée en ce que** l'ester hydrocarboné répond à la formule RCOOR' dans laquelle RCOO représente un reste d'acide gras comportant de 2 à 28 atomes de carbone et R' représente une chaîne hydrocarbonée contenant de 1 à 28 atomes de carbone.

5. Composition selon l'une quelconque des revendications 1 à 4, **caractérisée en ce que** l'ester hydrocarboné est en C₁₀ à C₂₅ et notamment en C₁₄ à C₂₂.

6. Composition selon l'une quelconque des revendications précédentes, **caractérisée en ce que** ledit ester est choisi parmi les esters de synthèse notamment d'acide gras comme l'huile de purcellin, le myristate d'isopropyle, le palmitate d'éthyle, le stéarate d'octyle ; les esters hydroxylés comme le lactate d'isostéaryle, l'hydroxystéarate d'octyle, l'adipate de diisopropyle, les heptanoates, octanoates, décanoates d'alcool gras, les esters de l'acide néopentanoïque comme le néopentanoate d'isodécyle, le néopentanoate d'isotridécyle, le néopentanoate d'isostéaryle, le néopentanoate d'octyldocécyle, les esters de l'acide isononanoïque comme l'isononanoate d'isononyle, l'isononanoate d'octyle, l'isononanoate d'isodécyle, l'isononanoate d'isotridécyle, l'isononanoate d'isostéaryle, les esters de l'alcool isopropylique, tels que le myristate d'isopropyle, le palmitate d'isopropyle, le stéarate ou l'isostéarate d'isopropyle, l'octanoate de cétyle, l'octanoate de tridécyle, le 4-diheptanoate et le palmitate d'éthyl-2-hexyle, le benzoate d'alkyle, le diheptanoate de polyéthylène glycol, le diéthyl 2- d'hexanoate de propylèneglycol et leurs mélanges.

7. Composition selon l'une quelconque des revendications précédentes, **caractérisée en ce que** ledit ester est l'isononanoate d'isononyle.

8. Composition selon l'une quelconque des revendications précédentes, **caractérisée en ce que** ledit ester est présent à raison de 5 à 90 % notamment de 10 à 60 %, et en particulier de 20 à 50 % en poids par rapport au poids total de la composition.

9. Composition selon l'une quelconque des revendications précédentes, **caractérisée en ce que** le radical hydrophile de l'organopolysiloxane est un groupement hydrocarboné saturé ou insaturé comprenant éventuellement un ou plusieurs hétéroatomes comme par exemple l'oxygène ou le soufre, et pouvant être substitué par au moins un groupement hydrophile.

10. Composition selon la revendication 9, **caractérisée en ce que** le groupement hydrophile est choisi parmi des fonctions hydroxyle, carboxylique, carboxylate, thiol, amine, sulfonate, sulfate, phosphate et/ou hydroxypolyéthylénoxy.

11. Composition selon l'une quelconque des revendications précédentes, **caractérisée en ce que** l'organopolysiloxane porte au moins un radical hydrophile, qui répond à la formule (II) : 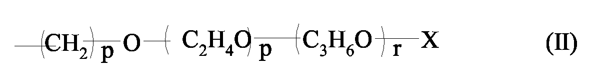 dans laquelle
- p varie de 0 à 5, q varie de 0 à 100, r varie de 0 à 50, avec p ou q étant différent de zéro,
- les unités (C₂H₄ O) et (C₃ H₆ O) peuvent être réparties aléatoirement ou par blocs, et
- X est un hydrogène ou un radical alkyle en C₁-C₁₀ le cas échéant substitué par un ou plusieurs fonctions de type hydroxyle, thiol, amine, carboxylique, carboxylate, amide, phosphate, sulfate et sulfonate.

12. Composition selon l'une quelconque des revendications précédentes, **caractérisée en ce que** l'organopolysiloxane comprend au moins un groupement hydroxy-polyéthylèneoxy.

13. Composition selon l'une quelconque des revendications précédentes, **caractérisée en ce que** l'organopolysiloxane répond à la formule générale (I) suivante : 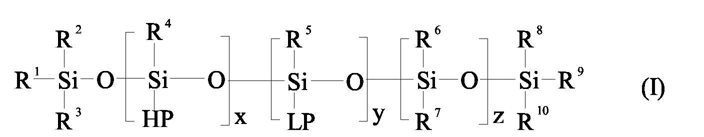 dans laquelle
- R¹, R², R³, R⁴, R⁵, R⁶ R⁷, R⁸, R⁹ et R¹⁰ représentent indépendamment l'un de l'autre un radical alkyle en C₁ à C₆, linéaire, ramifié ou cyclique, saturé ou non,
- HP est un radical portant au moins un groupement hydrophile,
- LP est un radical lipophile, et
- x varie de 1 à 5000 ; y de 0 à 5000 ; z de 0 à 5000.

14. Composition selon la revendication 13, **caractérisée en ce que** le groupement hydrophile répond à la définition selon la revendication 10.

15. Composition selon l'une quelconque des revendications précédentes, **caractérisée en ce que** l'organopolysiloxane appartient à la famille des diméthicone-polyéthylèneglycols.

16. Composition selon l'une quelconque des revendications précédentes, **caractérisée en ce que** l'organopolysiloxane est choisi parmi les diméthicones copolyol, en particulier le cétyl diméthicone copolyol et leurs dérivés.

17. Composition selon l'une quelconque des revendications précédentes, **caractérisée en ce que** l'organopolysiloxane peut être en tout ou partie, fluoré.

18. Composition selon l'une quelconque des revendications précédentes, **caractérisée en ce que** l'organopolysiloxane est présent à raison de 0,1 à 50 %, notamment de 0,5 à 40 %, et en particulier de 1 à 30 % en poids par rapport au poids total de la composition.

19. Composition selon l'une quelconque des revendications précédentes, **caractérisée en ce que** l'huile hydrocarbonée est apolaire.

20. Composition selon l'une quelconque des revendications précédentes, **caractérisée en ce que** l'huile hydrocarbonée est non volatile.

21. Composition selon l'une quelconque des revendications précédentes, **caractérisée en ce que** l'huile hydrocarbonée est choisie parmi les huiles d'origine animale, telle que le perhydrosqualène, les huiles hydrocarbonées végétales telles que les triglycérides liquides d'acide gras de 4 à 24 atomes de carbone comme les triglycérides heptanoïques et octanoïques, les huiles de tournesol, de maïs, de soja, de courge, de pépins de raisin, de sésame, de colza, de noisette, d'abricot, de macadamia, de ricin, d'avocat, les triglycérides des acides capryliques/capriques, l'huile de jojoba, le beurre de karité ; les hydrocarbures linéaires ou ramifiés d'origine minérale ou synthétique, tels que les huiles de paraffines et leurs dérivés, la vaseline, les polydécènes, les polyisobutènes hydrogénées, les huiles hydrocarbonées et/ou siliconées partiellement fluorées et leurs mélanges.

22. Composition selon l'une quelconque des revendications précédentes, **caractérisée en ce que** l'huile hydrocarbonée est une isoparaffine de type polyisobutène hydrogéné.

23. Composition selon l'une quelconque des revendications précédentes, **caractérisée en ce que** l'huile hydrocarbonée est présente à raison de 3,5 à 50 %, notamment de 4 à 40 %, et en particulier de 4 à 35 % en poids par rapport au poids total de la composition.

24. Composition selon l'une quelconque des revendications précédentes, **caractérisée en ce qu'**elle comprend en outre, au moins un corps gras différent de l'organopolysiloxane, de l'huile hydrocarbonée et de l'ester hydrocarboné et notamment choisi parmi les cires, les corps gras pâteux à température ambiante, les huiles cosmétiquement et dermatologiquement acceptables et leurs mélanges.

25. Composition selon l'une quelconque des revendications précédentes, **caractérisée en ce qu'**elle comprend en outre au moins une charge et/ou une matière colorante.

26. Composition selon l'une quelconque des revendications précédentes **caractérisée en ce qu'**elle se présente sous la forme d'un fond de teint, d'un fard à joues ou à paupières, d'un rouge à lèvres, d'une base ou baume de soin pour les lèvres, d'un produit anti-cernes, d'un eye-liner et/ou d'un mascara.

27. Procédé de soin cosmétique ou de maquillage des lèvres, de la peau, ou des phanères comprenant l'application sur les lèvres, la peau ou les phanères d'une composition selon l'une des revendications 1 à 26.

28. Utilisation d'une composition selon l'une quelconque des revendications 1 à 26 pour obtenir un maquillage de la peau, des lèvres ou des phanères.
